# EUROPEAN PATENT APPLICATION

(11) **EP 1 593 379 A2**
(43) Date of publication of application: **09.11.2005**
(21) Application number: 05076787.0
(22) Date of filing: 17.04.2000
(51) Int. Cl.: A61K 31/4439, A61P 11/06, A61P 17/06, A61P 17/08, A61P 17/10, A61P 17/16, A61P 19/02, A61P 19/08, A61P 9/10, A61P 13/12, A61P 31/18, A61P 37/06, A61P 37/08, A61P 35/02, A61P 3/10, A61P 21/04

(54) **Uses of PPAR-gamma agonists in neutrophil-induced diseases**

(30) Priority: 15.04.1999 GB 9908647
(62) Divisional of application: 00927425.9
(71) Applicant: SmithKline Beecham p.l.c., Brentford, Middlesex TW8 9GS (GB)
(72) Inventor: MacPhee, Colin Houston, GlaxoSmithKline, Park, Third Avenue Harlow Essex CM19 5AW (GB)
(74) Representative: Rutter, Keith

(57) **Abstract**

A method for the treatment of a disease or condition associated with increased numbers of neutrophils and/or neutrophil over-activation in a mammal such as a human, which method comprises administering an effective, non-toxic and pharmaceutically acceptable amount of a PPARγ agonist, such as 5-[4-[2-(N-methyl-N-(2-pyridyl)amino)ethoxy]benzyl]thiazolidine-2,4-dione, to a mammal in need thereof.

## Description

This invention relates to a method of treatment, in particular to a method for the treatment of diseases or conditions associated with increased numbers of neutrophils and/or over-activation of neutrophils.

European Patent Application, Publication Number 0,306,228 relates to certain thiazolidinedione derivatives disclosed as having antihyperglycaemic and hypolipidaemic activity. One particular thiazolidinedione disclosed in EP 0306228 is 5-[4-[2-(N-methyl-N-(2-pyridyl)amino)ethoxy]benzyl]thiazolidine-2,4-dione (hereinafter 'Compound (I)'). WO94/05659 discloses certain salts of Compound (I) including the maleate salt at example 1 thereof.

Compound (I) is an example of a class of anti-hyperglycaemic agents known as "glitazones" or "thiazolidinedione".

European Patent Applications, Publication Numbers: 0008203, 0139421, 0032128,0428312,0489663,0155845,0257781,0208420,0177353,0319189, 0332331, 0332332, 0528734, 0508740; International Patent Application, Publication Numbers 92/18501, 93/02079, 93/22445 and United States Patent Numbers 5104888 and 5478852, also disclose certain thiazolidinediones.

The contents of the above mentioned publications are incorporated herein by reference.

It is known that the γ-isoform of peroxisome proliferator-activated receptor (herein after PPARγ) is member of a nuclear receptor superfamily that includes receptors for the steroid, thyroid and retinoid hormones( Evans, Science 240, 889-895, (1988)). It is also known from Chawla *et al* that PPARγ is expressed early during the differentiation of adipocytes (Endocrinology 135,798-800, 1994).
It is known from J. Biol. Chem., 270,12963-12966 that thiazolidinediones such as Compound (I) are PPARγ agonists.

We have now demonstrated that PPARγ expression is significantly up-regulated in activated neutrophils. Thus, PPARγ agonists are expected to inhibit activated neutrophil function and therefor to have potential use in diseases and conditions where suppression of neutrophil activity or numbers would be of benefit. Such disorders include those listed herein including: gout, arthritis, asthma, chronic obstructive pulmonary disease, irritable bowel syndrome, psoriasis and acne.

Also neutrophils are a major cell type infiltrating the skin dermis and epidermis layer following moderate to server UV sun exposure. These cells have the capability to cause tissue damage and in certain severe circumstances delayed healing of the skin. Thus PPARγ agonists are considered to be useful in preventing and/or treating damage to the skin dermis and/or epidermis layers caused by excess ultra violet radiation or sun-light exposure. They are also considered to aid healing of the skin following such damage.

Accordingly, the invention provides a method for the treatment of a disease or condition associated with increased numbers of neutrophils and/or neutrophil over-activation in a mammal such as a human, which method comprises administering an effective, non-toxic and pharmaceutically acceptable amount of a PPARγ agonist, such as Compound (I), to a mammal in need thereof.

In an alternative aspect the invention provides a use of a PPARγ agonist, such as Compound (I), for the manufacture of a medicament for the treatment of a disease or condition associated with increased numbers of neutrophils and/or neutrophil over-activation in a mammal such as a human.

Suitable diseases or conditions associated with increased numbers of neutrophils and/or neutrophil over-activation include diseases or conditions of the respiratory tract, bone and joints, skin, gastrointestinal tract, other tissues and systematic diseases, allograft rejections and certain cancers. In particular a disease or of the respiratory tract. In particular a disease or condition of the bone and joints. In particular a disease or condition of the skin. In particular a disease or condition of the gastrointestinal tract. In particular a diseases or condition of other tissues and systematic diseases. In particular conditions associated with allograft rejections. In particular certain cancers.

Suitable diseases or conditions of the respiratory tract include those in the list consisting of: obstructive airways diseases including chronic obstructive pulmonary disease (COPD); asthma, such as bronchial, allergic, intrinsic, extrinsic and dust asthma, particularly chronic or inveterate asthma (eg late asthma and airways hyper-responsiveness); bronchitis; acute allergic, atrophic rhinitis and chronic rhinitis including rhinitis caseosa, hypertrophic rhinitis, rhinitis purulenta, rhinitis purulenta, rhinitis sicca and rhinitis medicarmenrosa; membranous rhinitis including croupous, fibrinous and pseudomembranous rhinitis and scrofoulous rhinitis; seasonal rhinitis including rhinitis nervosa (hay fever) and vasomotor rhinitis; sarcoiosis, farmer's lung and related diseases, fibrcic lung and idiopathic interstitial pneumonia. A particular disease or condition of the respiratory tract is considered to be each individual member of the preceeding list not including any other member of the said list.

Suitable diseases or conditions of the bone and joints include those in the list consisting of: rheumatoid arthritis, seronegative spondyloarthropathis (including ankylosing spondylitis, psoriatic arthritis and Reiter's disease), Behcet's disease, Sjogren's syndrome and systemic sclerosis. A particular disease or condition of the bone and joints is considered to be each individual member of the preceeding list not including any other member of the said list.

Suitable diseases or conditions of the skin include those in the list consisting of: psoriasis, atopical dermatitis, contact dermatitis and other eczmatous dermitides, seborrcetic dermatitis, Lichan planus, Pemphigus, bullous Pemphigus, Epidermolysis bullosa, urticaria, angiodermas, vasculitides, erythemas, cutaneous eosinophilias, uveitis, Alopecia areata and vemal conjunctivitis. A particular disease or condition of the skin is considered to be each individual member of the preceeding list not including any other member of the said list.

Suitable diseases or conditions of the gastrointestinal tract include those in the list consisting of: Coeliac disease, proctitis, eosinopillic gastro-enteritis, mastocytosis, Crohn'd disease, ulcerative colitis, food-related allergies which have effects remote from the gut e.g. migraine, rhinitis and eczema. A particular disease or condition of the gastrointestinal tract is considered to be each individual member of the preceeding list not including any other member of the said list.

Suitable diseases of other tissues and systematic diseases include those in the list consisting of: multiple sclerosis, atherosclerosis, Acquired Immundeficiency Syndrome (AIDS), lupus erythematosus, systemic lupus, erythtmatosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia pupura. A particular disease or condition of other tissues and systematic diseases is considered to be each individual member of the preceeding list not including any other member of the said list.

Suitable conditions associated with allograft rejection include those associated with the list consisting of: acute and chronic conditions following transplantation of kidney, heart, liver, lung, bone marrow, skin and cornea; and chronic graft versus host disease. A particular condition associated with allograft rejection is considered to be each individual member of the preceeding list not including any other member of the said list.

Suitable cancers include those in the list consisting of: non-small cell lung cancer (NSCLC) and squamous sarcoma. A particular cancer is non-small cell lung cancer (NSCLC). A particular cancer is squamous sarcoma

In a further aspect, there is included the conditions or diseases in the list consisting of: cystic fibrosis, stroke, re-perfusion injury in the heart, brain, peripheral limbs and sepsis, gout, arthritis, asthma, chronic obstructive pulmonary disease, irritable bowel syndrome, psoriasis and acne. A particular condition or disease is considered to be each individual member of the preceeding list not including any other member of the said list.

As indicated above, a further condition of the skin associated with increased numbers of neutrophils and/or neutrophil over-activation is damage to the skin dermis and/or epidermis layers caused by excess ultra violet radiation or sun-light exposure. Accordingly, the invention also provides a method for preventing and/or treating damage to the skin dermis and/or epidermis layers caused by excess ultra violet radiation or sun-light exposure in a mammal such as a human, which method comprises administration, preferably topical administration, of an effective, non-toxic and pharmaceutically acceptable amount of a PPARγ agonist, such as Compound (I), to a mammal in need thereof

Also provided is a method for promoting or aiding healing of the skin following such damage, which method comprises administration, preferably topical administration, of an effective, non-toxic and pharmaceutically acceptable amount of a PPARγ agonist, such as Compound (I), to a mammal in need thereof.

A suitable PPARγ agonist is Compound (I) or a pharmaceutically acceptable derivative thereof.

Other suitable PPARγ agonists include (+) -5-[[4-[(3,4-dihydro-6-hydroxy-2,5,7,8-tetramethyl-2H-1-benzopyran-2-yl)methoxy]phenyl]methyl]-2,4-thiazolidinedione (or troglitazone;
5-[4-[(1-methylcyclohexyl)methoxy]benzyl] thiazolidine-2,4-dione (or ciglitazone);5-[4-[2-(5-ethylpyridin-2-yl)ethoxy]benzyl] thiazolidine-2,4-dione (or pioglitazone); and 5-[(2-benzyl-2,3-dihydrobenzopyran)-5-ylmethyl)thiazolidine-2,4-dione (or englitazone); or a pharmaceutically acceptable derivative thereof.

It will be understood that the PPARγ agonist, such as Compound (I), is administered in a pharmaceutically acceptable form including pharmaceutically acceptable derivatives.

Suitable pharmaceutically derivatives include pharmaceutically acceptable salts, esters and solvates, as appropriate to the relevant PPARγ agonist.

Suitable pharmaceutically acceptable salted and or solvated forms of Compound (I) include those described in EP 0306228 and WO94/05659. A preferred pharmaceutically acceptable salt of Compound (I) is a maleate. A preferred pharmaceutically acceptable solvate of Compound (I)is a hydrate.

Other pharmaceutically acceptable derivatives are those disclosed in standard reference texts such as the British and US Pharmacopoeias, Remington's Pharmaceutical Sciences (Mack Publishing Co.) and Martindale The Extra Pharmacopoeia (London, The Pharmaceutical Press) or the above mentioned publications.

The PPARγ agonists may be prepared using known methods, for example those disclosed in the above mentioned publications.

Compound (I) and pharmaceutically acceptable forms thereof may be prepared using known methods, for example those disclosed in EP 0306228 and WO94/05659 publications.

Compound (I) may exist in one of several tautomeric forms, all of which are encompassed by the term Compound (I) as individual tautomeric forms or as mixtures thereof. Compound (I) contains a chiral carbon atom, and hence can exist in up to two stereoisomeric forms, the term Compound (I) encompasses all of these isomeric forms whether as individual isomers or as mixtures of isomers, including racemates.

As used herein the term 'pharmaceutically acceptable' embraces both human and veterinary use: for example the term 'pharmaceutically acceptable' embraces a veterinarily acceptable compound.

For the avoidance of doubt, when reference is made herein to scalar amounts, including mg amounts, of Compound (I) in a pharmaceutically acceptable form, the scalar amount referred to is made in respect of Compound (I) *per se:* For example 2 mg of Compound (I) in the form of the maleate salt is that amount of maleate salt which contains 2 mg of Compound (I).

Neutrophil activation may be demonstrated using conventional methodology such as that disclosed in Current Protocols in Immunology, Vol I, Suppl 1, Unit 6.12.3., the disclosure of which is incorporated herein by reference.

Neutrophils may be isolated from human blood as described in Current Protocols in Immunology Vol I, Suppl 1 Unit 7.23.1, the disclosure of which is incorporated herein by reference.

In the method of the invention, the active medicaments are preferably administered in pharmaceutical composition form. Accordingly, in one aspect the invention provides a pharmaceutical composition for use in the treatment of a disease or condition associated with increased numbers of neutrophils and/or neutrophil over-activation in a mammal, such as a human, which composition comprises a PPARγ agonist, such as Compound (I), and a pharmaceutically acceptable carrier therefor.

The compositions may be adapted for any suitable mode of administration, for example oral administration, parenteral administration, sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The compositions are preferably in a unit dosage form in an amount appropriate for the relevant daily dosage based upon such factors as the particular compound chosen and the nature and severity of the disease or condition.

Suitable dosages including unit dosages of the PPARγ agonist, such as Compound (I), include the known dosages and unit doses for these compounds as described or referred to in reference texts such as the British and US Pharmacopoeias, Remington's Pharmaceutical Sciences (Mack Publishing Co.), Martindale The Extra Pharmacopoeia (London, The Pharmaceutical Press) or the above mentioned publications.

In the treatment of the invention the PPARγ agonist may be administered in accordance with know regimens described or referred to in reference texts such as the those referred to above or in the above mentioned publications.

In the treatments the medicaments may be administered from 1 to 6 times a day, but most preferably 1 or 2 times per day.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the Compound (I)s suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions may contain from 0.1% to 99% by weight, preferably from 10-60% by weight, of the active material, depending upon the method of administration.

As indicated the PPARγ agonist can also be administered in a pharmaceutically acceptable form suitable for topical application.

Topical application of the PPARγ agonist is particularly suited to the treatment of diseases or conditions of the skin, including those in the list mentioned above and the prevention and/or treatment of damage to the skin dermis and/or epidermis layers caused by excess ultra violet radiation or sun-light exposure. Psoriasis is a particular diseases or conditions of the skin.

Accordingly, in a further aspect, the invention also provides a pharmaceutical composition adapted for topical administration, especially for the treatment of diseases or conditions of the skin, comprising a PPARγ agonist and a pharmaceutically acceptable carrier.

Suitable pharmaceutically acceptable carriers adapted for topical application include any substance compatible with the active compound which can safely be used for topical or transdermal administration to human or non-human mammals and includes solvents, diluents, preservatives, penetration enhancers, polymers, buffers, perfumes, thickeners, gelling agents, surfactants and emulsifiers. Such substances are disclosed in standard reference texts such as the British and US Pharmacopoeias, Remington's Pharmaceutical Sciences (Mack Publishing Co.), Martindale The Extra Pharmacopoeia (London, The Pharmaceutical Press) Harry's Cosmeticology (Leonard Hill Books) and Drugs and Pharmaceutical Sciences, Vol 18, Dermatological Formulations " Percutaneous Absorption" by Brian W Barry, pub Marcel Deker (ISBN 0/8247/1729/5).or the above mentioned publications.

The active ingredient may comprise, for topical administration, from 0.001% to 10% w/w, for instance from 1% to 2% by weight of the formulation. It may however comprise as much as 10% w/w but preferably will comprise less than 5% w/w, more preferably from 0.1 % to 1% w/w of the formulation.

The compositions of the invention adapted for topical administration may be formulated in any convenient dosage form suitable for topical application, for example as a lotion, liniment, gel, cream, ointment, drops, solution or spray using methods disclosed in the reference texts mentioned above.

Lotions or liniments for application to the skin may also include an agent to hasten drying and to cool the skin, such as an alcohol or acetone, and/or a moisturizer such as glycerol or an oil such as castor oil or arachis oil.

Creams, ointments or pastes according to the present invention are semi-solid formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy base. The base may comprise hydrocarbons such as hard, soft or liquid paraffin, glycerol, beeswax, a metallic soap; a mucilage; an oil of natural origin such as almond, corn, arachis, castor or olive oil; wool fat or its derivatives or a fatty acid such as steric or oleic acid together with an alcohol such as propylene glycol or a macrogel. The formulation may incorporate any suitable surface active agent such as an anionic, cationic or non-ionic surfactant such as a sorbitan ester or a polyoxyethylene derivative thereof. Suspending agents such as natural gums, cellulose derivatives or inorganic materials such as silicaceous silicas, and other ingredients such as lanolin, may also be included.

Drops according to the present invention may comprise sterile aqueous or oily solutions or suspensions and may be prepared by dissolving the active ingredient in a suitable aqueous solution of a bactericidal and/or fungicidal agent and/or any other suitable preservative, and preferably including a surface active agent. The resulting solution may then be clarified by filtration, transferred to a suitable container which is then sealed and sterilized by autoclaving or maintaining at 98-100°C. for half an hour. Alternatively, the solution may be sterilized by filtration and transferred to the container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

In the topical treatments the medicaments may be applied from 1 to 6 times a day, but most preferably 1 or 2 times per day.

The present invention also provides a pharmaceutical composition adapted for topical administration comprising a PPARγ agonist and a pharmaceutically acceptable carrier adapted for topical application, for use as an active therapeutic substance.

Compositions may, if desired, be in the form of a pack accompanied by written or printed instructions for use.

The compositions are formulated according to conventional methods, such as those disclosed in standard reference texts, for example the British and US Pharmacopoeias, Remington's Pharmaceutical Sciences (Mack Publishing Co.) and Martindale The Extra Pharmacopoeia (London The Pharmaceutical Press) (for example see the 31 st Edition page 341 and pages cited therein) and Harry's Cosmeticology (Leonard Hill Books) or as disclosed in the above-mentioned publications.

No adverse toxicological effects have been established for the compositions or methods of the invention in the above mentioned dosage ranges.

The following example illustrates the invention but does not limit it in any way.

### DEMONSTRATION OF EFFECT

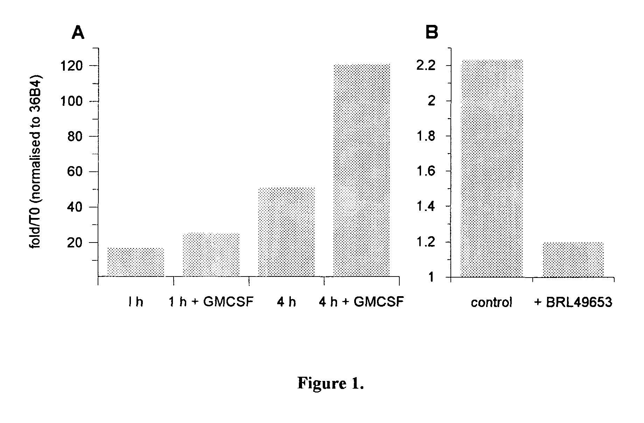

The results shown in Figure 1 demonstrate:
(Figure 1A): That PPARgamma mRNA is rapidly and significantly increased during (data taken at 1 and 4 hours) human neutrophil attachment to culture dishes and is further enhanced by treatment with GM-CSF, (Figure 1A).
(Figure 1B): That the increase in Gelatinase B mRNA expression observed 4 hours after neutrophil attachment was reduced by treatment with 1 µM of Compound (I). Time zero (T0) refers to expression in naïve human neutrophils prior to attachment.

### Figure 2: Inhibition of MMP-9 release by Compound (I)

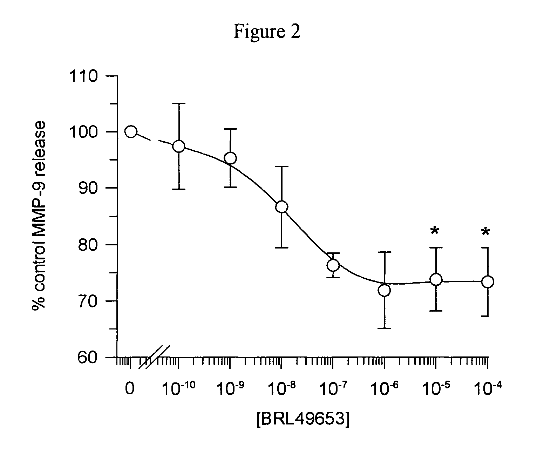

Neutrophils were isolated by countercurrent centrifugal elutriation to a purity of at least 98 % and were maintained in RPMI 1640 plus 1 % BSA. Cells were cultured in 48 well plates and attached to the wells within 1 hour of plating. Compound (I) (referred to as "BRL49653" in Figure 2) was included from the beginning of the experiment. After 4 hours the media was removed and a human MMP-9 ELISA assay (Amersham) was performed. Data (from 4 different individuals) were not suitable for pooling directly, so for the purposes of the figure they were normalised to percentage of no compound control. Asterisks denote p < 0.05 using a paired Student's t-test performed on the raw data prior to normalisation. IC50 for the inhibition by Compound (I) of MMP-9 release was 10.6 nM (calculated by fitting a 4-parameter logistic curve using Statistica).

## Claims

1. A method for the treatment of a disease or condition associated with increased numbers of neutrophils and/or neutrophil over-activation in a mammal such as a human, which method comprises administering an effective, non-toxic and pharmaceutically acceptable amount of a PPARγ agonist, such as Compound (I), to a mammal in need thereof.

2. A method according to claim 1, wherein the diseases or conditions associated with increased numbers of neutrophils and/or neutrophil over-activation is selected from: diseases or conditions of the respiratory tract, bone and joints, skin, gastrointestinal tract, other tissues and systematic diseases, allograft rejections and certain cancers.

3. A method according to claim 1 or claim 2, wherein the diseases or conditions of the respiratory tract are those in the list consisting of obstructive airways diseases including chronic obstructive pulmonary disease (COPD); asthma, such as bronchial, allergic, intrinsic, extrinsic and dust asthma, particularly chronic or inveterate asthma; bronchitis; acute allergic, atrophic rhinitis and chronic rhinitis including rhinitis caseosa, hypertrophic rhinitis, rhinitis purulenta, rhinitis purulenta, rhinitis sicca and rhinitis medicarmenrosa; membranous rhinitis including croupous, fibrinous and pseudomembranous rhinitis and scrofoulous rhinitis; seasonal rhinitis including rhinitis nervosa (hay fever) and vasomotor rhinitis; sarcoiosis, farmer's lung and related diseases, fibricic lung and idiopathic interstitial pneumonia.

4. A method according to any one of claims 1 to 3, wherein the diseases or conditions of the bone and joints are those in the list consisting of: rheumatoid arthritis, seronegative spondyloarthropathis, Behcet's disease, Sjogren's syndrome and systemic sclerosis.

5. A method according to any one of claims 1 to 3, wherein the diseases or conditions of the skin are those in the list consisting of: psoriasis, atopical dermatitis, contact dermatitis and other eczmatous dermitides, seborrcetic dermatitis, Lichan planus, Pemphigus, bullous Pemphigus, Epidermolysis bullosa, urticaria, angiodermas, vasculitides, erythemas, cutaneous eosinophilias, uveitis, Alopecia areata and vemal conjunctivitis.

6. A method according to any one of claims 1 to 3, wherein the diseases or conditions of the gastrointestinal tract are those in the list consisting of: Coeliac disease, proctitis, eosinopillic gastro-enteritis, mastocytosis, Crohn'd disease, ulcerative colitis and food-related allergies which have effects remote from the gut.

7. A method according to any one of claims 1 to 3, wherein the diseases of other tissues and systematic diseases are those in the list consisting of: multiple sclerosis, atherosclerosis, Acquired Immundeficiency Syndrome (AIDS), lupus erythematosus, systemic lupus, erythtmatosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia pupura.

8. A method according to any one of claims 1 to 3, wherein the conditions associated with allograft rejection are those associated with the list consisting of: acute and chronic conditions following transplantation of kidney, heart, liver, lung, bone marrow, skin and cornea; and chronic graft versus host disease.

9. A method according to any one of claims 1 to 3, wherein the cancers are those in the list consisting of: non-small cell lung cancer (NSCLC) and squamous sarcoma.

10. A method according to any one of claims 1 to 9, wherein the PPARγ agonist is 5-[4-[2-(N-methyl-N-(2-pyridyl)amino)ethoxy]benzyl]thiazolidine-2,4-dione ("Compound (I)") or a pharmaceutically acceptable derivative thereof.

11. A method according to any one of claims 1 to 9, wherein the PPARγ agonist 5-[4-[2-(5-ethylpyridin-2-yl)ethoxy]benzyl] thiazolidine-2,4-dione (or pioglitazone).
